# DEMANDE DE BREVET EUROPEEN

(11) **EP 4 566 544 A1**
(43) Date de publication de la demande: **11.06.2025**
(21) Numéro de dépôt: 24217546.1
(22) Date de dépôt: 04.12.2024
(51) Int. Cl.: A61B 7/04

(54) **STÉTHOSCOPE ÉLECTRONIQUE**

(30) Priorité: 05.12.2023 FR 2313606
(71) Demandeur: Withings, 92130 Issy-Les-Moulineaux (FR)
(72) Inventeur: Bedetti, Thomas, 92130 Issy-les-Moulineaux (FR); Merlot, Antoine, 92130 Issy-les-Moulineaux (FR); Tucoulat, Benoit, Issy-les-Moulineaux (FR); Barbedette, Thibaut, 92130 Issy-les-Moulineaux (FR); Droze, Thomas, 92130 Issy-les-Moulineaux (FR)
(74) Mandataire: Withings IP

(57) **Abrégé**

Il est proposé un dispositif portable de mesure physiologique (700) préhensible par un manipulateur comprenant :
- un boîtier de forme allongée selon une direction d'extension et comprenant, selon la direction d'extension, une extrémité,
- un stéthoscope (106R) positionné au niveau de l'extrémité et comprenant une membrane s'étendant dans un plan de membrane,
- un affichage (112), arrangé sur le boîtier, l'affichage (112) s'étendant selon une direction d'extension orthogonal au plan de membrane,
- une interface physique, reliée fonctionnellement à l'affichage (112) et positionnée sur le boîtier entre l'affichage (112) et la membrane le long de la direction d'extension.

## Description

### Domaine technique

La présente invention se rapporte aux dispositifs portables de mesures physiologiques (ci-après le dispositif de mesure), de type dispositif personnel tenable à la main (ou en anglais « *personal hand-held monitor* », PHHM) et incorporant une pluralité de capteurs de données physiologiques. De tels dispositifs sont notamment utilisables dans le cadre d'un suivi à distance, par exemple par un médecin au cours d'une téléconsultation ou au cours d'une consultions asynchrone. On parle plutôt généralement, en terminologie anglaise de « *telehealth* » ("télé-santé" en français) ou de « *remote patient monitoring* » (RPM, ou "suivi de patient à distance" en français). Dans le cadre de la description, le terme de RPM sera utilisé.

Une des particularités de ces dispositifs est qu'ils sont opérables en automesure, c'est-à-dire que la personne qui tient le dispositif est la personne qui est en train de subir les mesures.

Les mesures effectuées sont des mesures physiologiques d'un utilisateur qui tient à la main le dispositif de mesure. Ces mesures sont représentatives d'un état de santé de l'utilisateur.

### Technique antérieure

De nombreux dispositifs de mesure existent. Certains sont capables d'effectuer une seule mesure : thermomètre, stéthoscope, ECG, etc., tandis que d'autres sont capables d'effectuer plusieurs de ces mesures.

Parmi les stéthoscopes électroniques, on peut citer ceux décrits dans les documents suivants : US9265478, US20220354451, US11741931, US20220031256, US20010030077, US20230142937, US5737429A, US5638453A. Toutefois, tous ces documents ne fournissent pas des dispositifs adaptés pour l'auto-mesure et certains sont mêmes explicitement conçus pour être tenus par un médecin. De plus, le nombre de mesures peut être insuffisant pour avoir un dispositif adapté au RPM.

Parmi les thermomètres, on peut citer celui décrit dans le document WO2017114923A2 (au nom de Withings^{™}).

Le document US2022035445 au nom de EKO HEALTH^{™} et plus généralement toutes les publications de cette société décrivent un stéthoscope électronique incorporant un électrocardiogramme.

D'autres documents décrivent des dispositifs capables de faire deux ou trois mesures.

Par exemple, le document PL423977A1 décrit un stéthoscope avec un capteur infrarouge, mais sans détailler l'intégration.

Par exemple, le document WO2022200743 décrit un dispositif avec un boîtier parallélépipédique qui intègre un stéthoscope, un oxymètre, un otoscope et un thermomètre, en plus d'une brassière pour la mesure de pression.

Par exemple, le document US20200015774 décrit un dispositif avec une poignée circulaire pour mettre une base au contact d'un utilisateur. La base comprend un stéthoscope électronique qui peut aussi inclure un thermomètre ou un oxymètre. La divulgation fait aussi mention, sans aucun détail, de captation de signaux ECG et ultrasons.

Par exemple, le document WO201704463 décrit un dispositif à porter au doigt pour oxymétrie et qui intègre un stéthoscope et un otoscope.

Par exemple, le document WO2022253723 décrit un dispositif essentiellement cylindrique avec deux faces opposées qui comprennent trois capteurs physiologiques : thermomètre, stéthoscope et oxymètre.

Par exemple, le produit Linktop 6-in-1^{™} propose un dispositif en forme de mini-dalle carré, présentant un capteur de température et une électrode sur une même face latérale, un capteur optique et une électrode sur la face supérieure carré, et une électrode sur la face inférieure carré.

Néanmoins, ces documents présentent des limitations. Une limitation réside notamment dans la transition entre une idée conceptuelle et un produit fonctionnel sur le marché. Une autre limitation réside dans le nombre de mesures réalisables par chaque dispositif, une ou deux mesures, quelques rare fois trois mesures. Pour avoir un dispositif de RPM efficace, il est primordial de maximiser le nombre de mesures sur un même dispositif. Une autre limitation réside dans l'ergonomie et la praticité d'utilisation, à plus forte raison lorsque le nombre de mesures disponibles augmentent. En effet, le dispositif de mesure doit rester petit et simple à manipuler par les utilisateurs qui peuvent être âgés et en mauvaise santé. Ces contraintes techniques ont des conséquences directes sur l'adoption et la rétention par les utilisateurs.

### Résumé

La description se rapporte à des dispositifs de mesure offrant une praticité d'utilisation améliorée. Cette praticité peut concerner l'ergonomie. Cette praticité peut concerner le nombre de mesures permises par le dispositif.

Plus particulièrement, l'invention est définie dans les revendications.

Selon un aspect de la description, il est proposé un dispositif portable de mesure physiologique préhensible par un manipulateur comprenant :
- un boîtier de forme allongée selon une direction d'extension et comprenant, selon la direction d'extension, une extrémité,
- un stéthoscope électronique positionné au niveau de l'extrémité et comprenant une membrane s'étendant dans un plan de membrane,
- un affichage, arrangé sur le boîtier, l'affichage s'étendant selon une direction d'extension orthogonal au plan de membrane,
- une interface physique, reliée fonctionnellement à l'affichage et positionnée sur le boîtier entre l'affichage et la membrane le long de la direction d'extension.

Grâce à cet arrangement, l'utilisateur, qui est aussi manipulateur, peut facilement utiliser le stéthoscope sur lui-même, après avoir lancé une mesure sur l'affichage avec l'interface physique. De plus, l'écran se retrouve plus loin du corps, ce qui facilite la lecture. Enfin, il suffit d'effectuer une rapide translation du dispositif depuis une position de navigation à une main à une position de mesure à une main.

Dans un mode de réalisation, l'affichage est configuré pour afficher des informations lisibles depuis un sens transversal à la direction d'extension.

Dans un mode de réalisation, l'extrémité définit un bord et la membrane est inscrite dans le bord au niveau de l'extrémité.

Dans un mode de réalisation, le plan de membrane s'étend orthogonalement à la direction d'extension.

Dans un mode de réalisation, le dispositif comprend une face, dite face avant, et dans lequel l'affichage et l'interface physique sont positionnés sur la face avant.

Dans un mode de réalisation, l'extrémité définit un bord, l'interface physique étant positionnée entre le bord et la moitié, voire un tiers, d'une longueur du dispositif le long de la direction d'extension, depuis le bord.

Dans un mode de réalisation, l'extrémité est une deuxième extrémité et le boîtier comprend une première extrémité opposée à la deuxième extrémité le long de la direction d'extension, le dispositif comprenant une forme essentiellement parallélépipédique avec une face avant et une face arrière entre la première extrémité et la deuxième extrémité, dans lequel l'affichage et l'interface physique sont positionnés sur la face avant.

Dans un mode de réalisation, le dispositif comprend en outre un capteur d'extrémité disposé au niveau de la première extrémité.

Dans un mode de réalisation, l'affichage est configuré pour afficher des indications relatives au placement du stéthoscope sur le corps.

La description concerne également un procédé d'utilisation du dispositif tel que défini ci-dessus, le procédé comprenant au moins les étapes suivantes :
- utilisation de l'interface physique du dispositif dans une position de navigation manipulation à une main,
- mesure avec le stéthoscope dans une position de manipulation à une main.

### Brève description des dessins

D'autres caractéristiques, détails et avantages apparaîtront à la lecture de la description détaillée ci-après, et à l'analyse des dessins annexés, sur lesquels :
**Fig. 1****.**

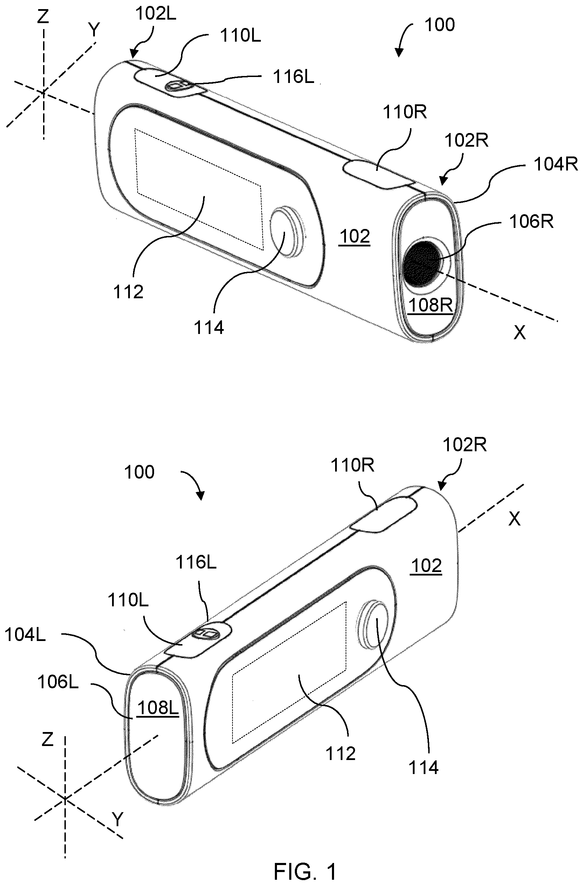

   [Fig. 1] Cette figure représente deux vues en 3D, côté droit et gauche, d'un dispositif de mesure selon un mode de réalisation.
**Fig.** 2

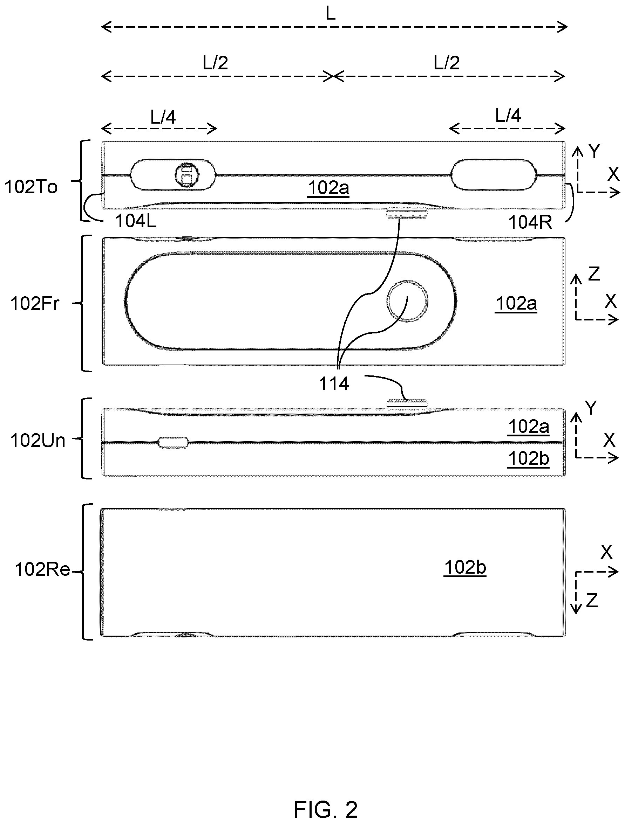

   [Fig. 2] Cette figure représente quatre vues projetées du dispositif de la figure 1.
**Fig. 3**

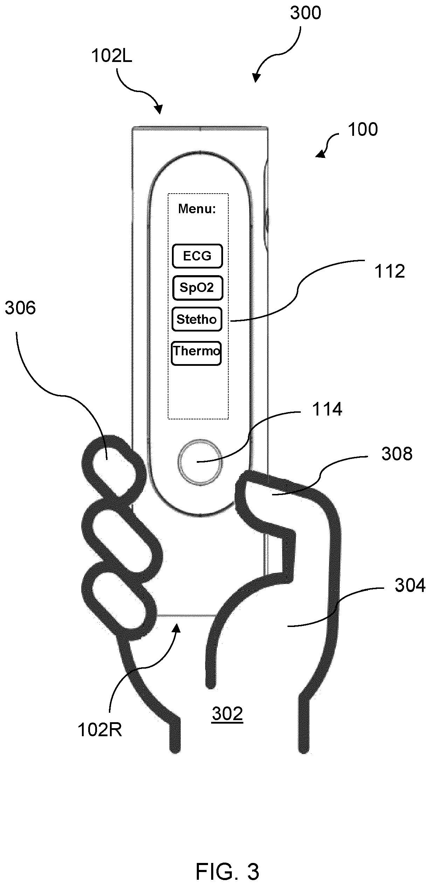

   [Fig. 3] Cette figure représente une vue du dispositif des figures 1 à 2, lors d'une manipulation en position de navigation, à une main.
**Fig. 4**

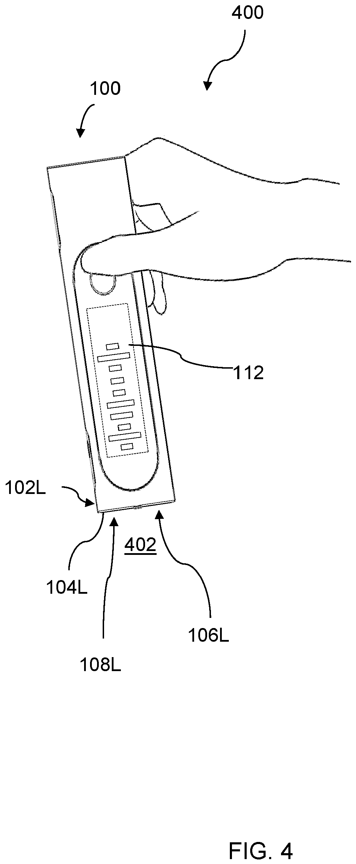

   [Fig. 4] Cette figure représente une vue du dispositif des figures 1 à 3, lors d'une manipulation en position à une main pour un premier capteur du dispositif.
**Fig. 5**

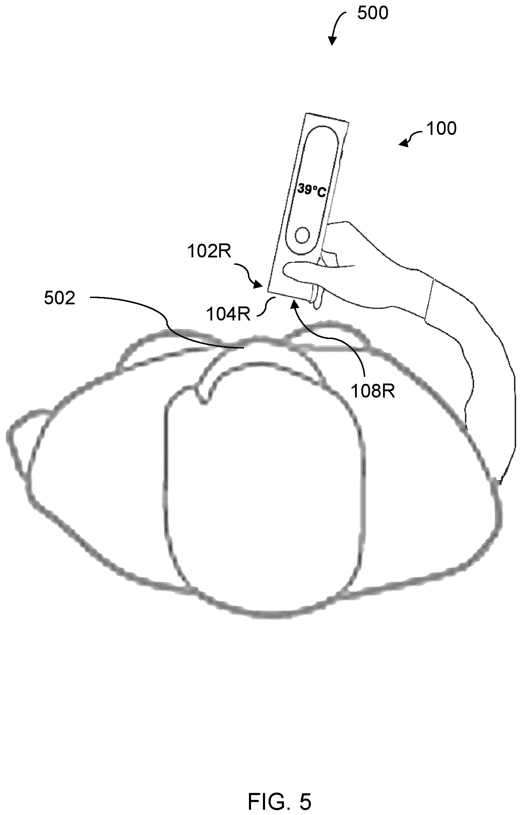

   [Fig. 5] Cette figure représente une vue du dispositif des figures 1 à 4, lors d'une manipulation en position à une main pour un deuxième capteur du dispositif.
**Fig. 6**

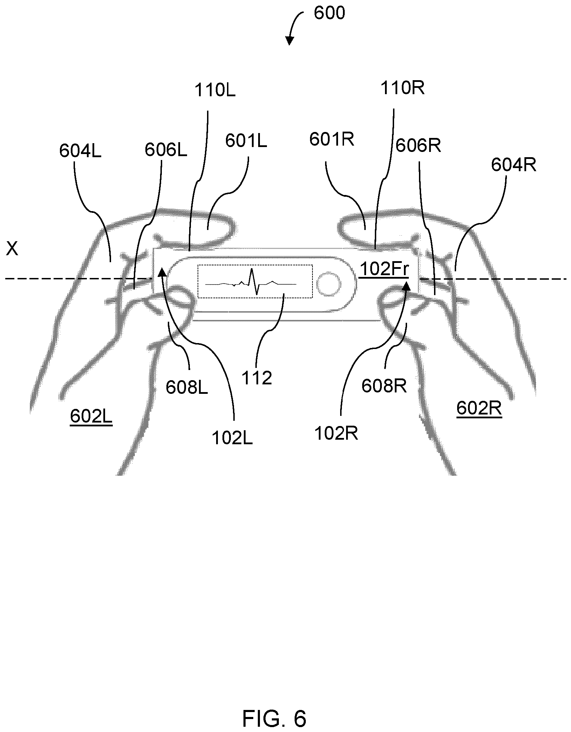

   [Fig. 6] Cette figure représente une vue du dispositif des figures 1 à 5, lors d'une manipulation en position à deux mains pour un capteur de doigt du dispositif.
**Fig. 7**

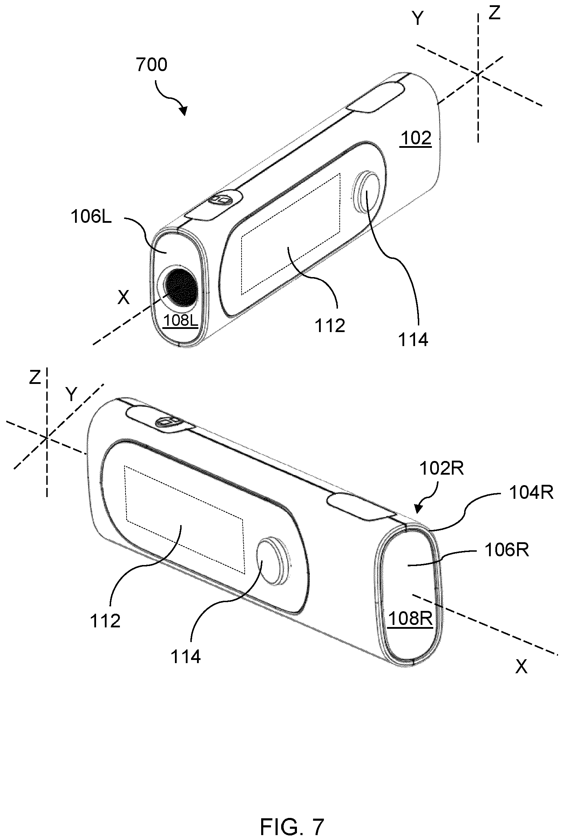

   [Fig. 7] Cette figure représente deux vues en 3D, côté droit et gauche, d'un dispositif de mesure selon un autre mode de réalisation.
**Fig. 8**

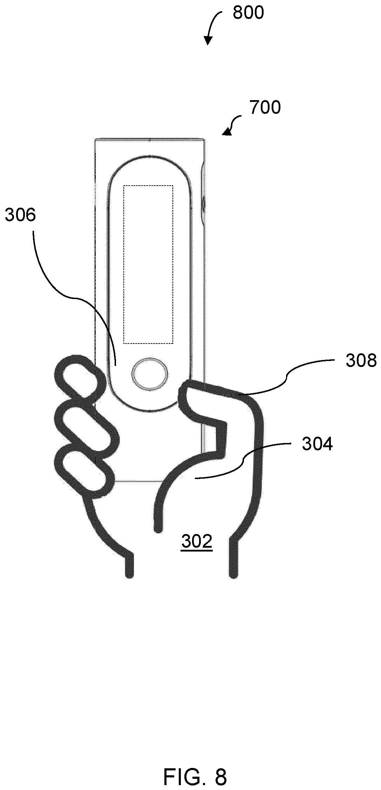

   [Fig. 8] Cette figure représente une vue du dispositif de la figure 7, lors d'une manipulation en position de navigation, à une main.
**Fig. 9**

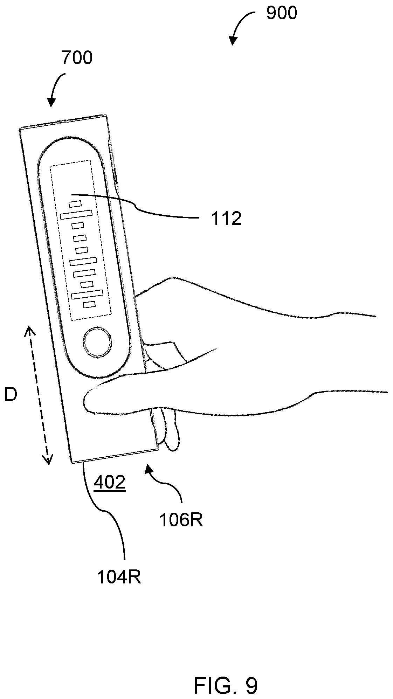

   [Fig. 9] Cette figure représente une vue du dispositif des figures 7 à 8, lors d'une manipulation en position à une main pour un deuxième capteur du dispositif.
**Fig. 10**

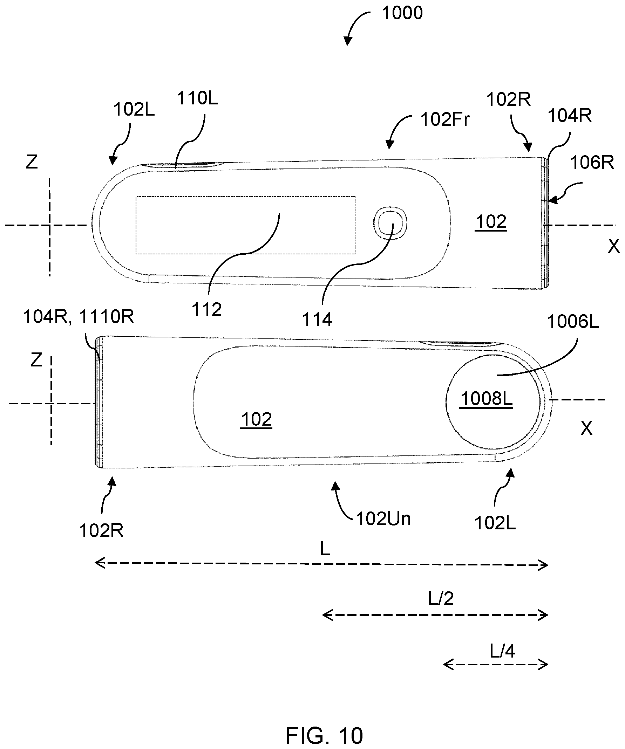

   [Fig. 10] Cette figure représente deux vues projetées, face avant et arrière, d'un dispositif de mesure selon un autre mode de réalisation.
**Fig. 11**

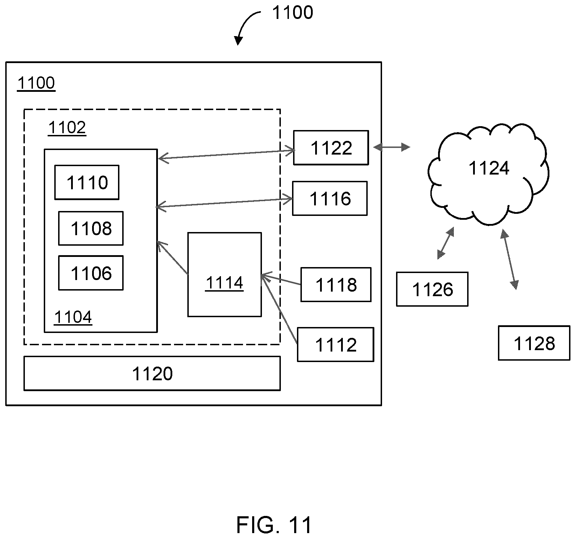

   [Fig. 11] Cette figure représente une vue schématique des dispositifs et de leur environnement.

### Description détaillée

### Présentation générale

La présente description va décrire plusieurs modes de réalisation et variantes des dispositifs de mesures physiologiques qui sont portables, préhensibles par une ou deux mains et adapté aux automesures. On parlera de « dispositif » pour simplifier le langage. Par définition, le manipulateur est celui qui tient le dispositif et l'utilisateur est celui qui subit la mesure. Dans le cadre de cette description, sauf mention contraire, manipulateur et utilisateur sont confondus.

Le dispositif intègre un ou plusieurs capteurs physiologiques pour mesurer des caractéristiques physiologiques (« mesure physiologique ») d'un utilisateur qui est aussi le manipulateur. A cet égard, le capteur peut être l'un parmi : un capteur de température pour mesurer la température corporelle de l'utilisateur, un capteur de son pour mesurer les sons du coeur ou des poumons, un capteur ECG (électrocardiogramme) pour mesurer les pulsations et le rythme du coeur, un capteur d'impédance, un capteur optique, de type PPG (pour « PhotoPléthysmoGraphie ») par exemple, pour de l'oxymétrie, pour mesurer la saturation en oxygène du sang (SpO2) ou d'autres grandeurs telles que le rythme cardiaques, un capteur de force, pour notamment mesurer une force ou une pression relative au doigt (in fine pour déterminer une pression artérielle), un spiromètre, etc.

Par mesure physiologique, il est signifié des mesures de caractéristique physiologique d'un utilisateur ou utilisatrice (on parlera d'utilisateur par la suite), qui traduisent un état de santé, telles que : la température, les sons du coeur, les sons des poumons, le rythme cardiaque, l'arythmie, etc. En particulier, dans un mode de réalisation, le dispositif intègre au moins deux capteurs physiologiques ; dans un autre mode de réalisation, le dispositif intègre au moins trois capteurs physiologiques (par exemple : thermomètre, stéthoscope, ECG) ; dans un autre mode de réalisation, e dispositif intègre au moins quatre capteurs physiologiques (par exemple : thermomètre, stéthoscope, ECG, PPG).

Par portable, il est signifié un dispositif qui est léger et peu encombrant. Le dispositif peut donc être saisi aisément à une main par un utilisateur et le dispositif peut par exemple être rangé facilement dans un tiroir, un sac à main ou une poche de pantalon. A titre d'exemple, le dispositif pèse moins de 250g, voire 150g. A titre d'exemple, le dispositif présente un volume inférieur à 20x5x10 cm, voire 18x3x5cm, voire encore 15x2,5x4cm.

Le dispositif présente une forme allongée selon une direction d'extension, par exemple une forme essentiellement parallélépipédique.

De plus, le dispositif peut être connecté, en ce sens qu'il peut envoyer des données vers un appareil tiers, tel qu'un smartphone ou un serveur. Cette connectivité permet au dispositif de fonctionner comme un dispositif de RPM, l'utilisateur devenant alors un patient d'un médecin à distance. La téléconsultation peut se faire en synchrone, avec une interaction en direct ou quasi-direct avec le médecin ou bien en asynchrone. En synchrone, le patient utilise le dispositif pour acquérir des données physiologiques qui sont immédiatement ou quasi-immédiatement transmises au médecin (quelques secondes après). En asynchrone, le patient utilise le dispositif lorsqu'il le peut et le médecin consulte les données physiologiques lorsqu'il le peut, c'est-à-dire plus tard temporellement.

La forme du boîtier et l'arrangement du ou des capteurs dans le dispositif est prévu pour que ce dernier soit aisément utilisable à une main pour effectuer certaines mesures physiologiques (par exemple : température et son du coeur ou des poumons) et à deux mains pour effectuer d'autres mesures physiologiques (par exemple ECG ou PPG). Dans un mode de réalisation, la position à une main peut s'apparenter à une position de télécommande, pour laquelle les usagers sont généralement familiers, et la position à deux mains peut s'apparenter à une position de manette de console de jeux vidéo, pour laquelle les usagers sont globalement familiers aussi.

Le dispositif est destiné à être utilisé en automesure. Il doit donc pouvoir être facilement utilisé par un utilisateur pour qu'il puisse effectuer des mesures sur lui-même.

Toutefois, le dispositif doit aussi pouvoir être utilisé à deux, par exemple lorsqu'une personne est en incapacité de manipuler le dispositif (handicap ou enfant par exemple). Ainsi, les contraintes liées à la condition d'utilisation seul ne doivent pas générer des contraintes d'utilisation à plusieurs.

La figure 1 représente deux vues tridimensionnelles d'un dispositif 100 selon un mode de réalisation. La figure 2 illustre le dispositif 100 selon quatre projections. La figure 3 représente une vue du dispositif 100 lors d'une manipulation en position de navigation, à une main. Les figures 4 et 5 illustrent chacune une vue du dispositif 100 lors d'une manipulation en position de mesure à une main (pour deux mesures différentes). La figure 6 illustre une vue du dispositif 100 lors d'une manipulation en position de mesure à deux mains.

### Le boîtier

Le dispositif 100 comprend un boîtier 102 de forme allongée qui définit une direction d'extension X. Le boitier 102 définit également deux directions transversales orthogonales Y et Z. Par la suite, les notions de « transversal » sont définies par rapport à la direction X. Cette direction d'extension X est dite principale car le dispositif 100 présente sa plus grande dimension selon cette direction. La direction d'extension X est rectiligne sur les figures mais une courbure est possible dans la mesure où la manipulation du dispositif ne serait pas altérée significativement.

Le long de la direction d'extension X, le boîtier 102 comprend une première extrémité 102L (L pour « Left » ou gauche en français) et une deuxième extrémité 102R (R pour « Right » ou droit en français), opposé à la première extrémité 102L. La première extrémité 102L définit un premier bord 104L et la deuxième extrémité 102R définit un deuxième bord 104R. Chaque bord 104L, 104R définit une courbe fermée (de forme ovoïde sur les figures du fait de la section du boîtier 102 au niveau de la première extrémité 102L et de la deuxième extrémité 102R).

Pour permettre une préhension aisée, chaque bord 104L, 104R a une longueur inférieure à 30cm, voire à 15cm. La description présentera en détail les différentes positions de manipulation possible du dispositif 100 par un utilisateur.

Dans un mode de réalisation, la distance L entre les deux bords 104L, 104R, le long de la direction d'extension X est inférieur à 20cm, voire 15cm. Cette distance L permet de garantir le caractère portable du dispositif 100.

Dans un mode de réalisation, la distance L entre les deux bords 104L, 104R, le long de la direction d'extension X est supérieure à 8cm, voire 10cm. Cette distance L permet de garantir que le caractère préhensible à une main et deux mains. En effet, un dispositif trop petit ne permet pas d'être manipulé aisément, notamment par tout le monde.

Le dispositif 100 est conçu pour être saisi par au moins une des deux extrémités 102L, 102R avec la main dans le prolongement du boîtier 102 selon la direction longitudinale. En particulier, on définit une position de navigation, illustrée en figure 3.

Le boîtier 102 peut avoir une forme essentiellement cylindrique, avec une section transversale dans le plan YZ (i.e. orthogonale à la direction d'extension X) de forme convexe. Dans un mode de réalisation, cette section présente deux axes de symétrie, par exemple les axes Y et Z tels que représentés sur les figures. La section est par exemple une section oblongue, comme illustré sur les figures, ou bien une section rectangulaire (avec des angles plus ou moins arrondis), ou bien une section circulaire.

Par essentiellement cylindrique, il est signifié que la section orthogonale à la direction d'extension X ne présente pas de variation de dimension significative (par exemple moins de 20% de variation de diamètre maximum).

Le boîtier 102 est typiquement en matériau plastique, pour être léger, économique et isolant électriquement. Lorsque l'utilisateur tient le dispositif 100, sa ou ses mains sont majoritairement en contact avec le boîtier 102. Le boîtier 102 peut être formé de plusieurs pièces assemblées entre elle. Sur les figures 1 et 2, le boîtier 102 comprend deux coques, notées respectivement 102a, 102b, qui peuvent être assemblées au niveau d'une jonction parallèle à la direction d'extension X. En variante, le boitier 102 est formé d'une coque unique. D'autres types d'assemblage sont possibles.

Au moins deux faces reliant le bord 104L au bord 104R peuvent être définies pour le boîtier 102. Dans le cas d'une section oblongue ou rectangulaire, on définit pour le dispositif, comme illustré sur la figure 2, une face avant 102Fr (Fr pour "Front" ou avant en français), une face arrière 102Re (Re pour "Rear" ou arrière en français) (opposée à la face avant), une face supérieure 102To (To pour "Top" ou haut en français), et une face inférieure 102Un (Un pour "Under" ou bas en français), opposée à la face supérieure ; enfin les deux bords 104L, 104R définissent chacun une face latérale. Les faces avant 102Fr et arrière 102Re sont reliées par la face supérieure 102To et la face inférieure 102Un. En particulier, la face avant 102Fr et la face supérieure 102To sont perpendiculaire ou essentiellement perpendiculaire entre elles (en négligeant les arrondis de forme possible tels que visibles en figure 1).

Ces noms de face sont définis par rapport à la position de manipulation à deux mains illustrées en figure 6.

Comme illustré sur la figure 2, chacune des faces supérieure 102To et inférieure 102Un s'étend principalement dans un plan longitudinal XY. Chacune des faces avant 102Fr et arrière 102Re s'étend principalement dans un plan longitudinal XZ, orthogonal au plan XY. Chacun des deux bords 104L, 104R s'étend principalement dans un plan transversal XZ, c'est-à-dire que les faces latérales sont aussi, sur les figures, orthogonales à la direction d'extension X. Des formes arrondies pour les faces avant 102Fr, arrière 102Re, supérieure 102To et inférieure 102Un peuvent être prévues, comme illustrées sur les figures, pour ne pas avoir d'arête ou ainsi faciliter la préhension.

Les faces avant 102Fr et arrière 102Re ont une hauteur (la dimension en Z) plus important que la profondeur (la dimension en Y) des faces arrière 102Re et supérieure 102To. Dit autrement, le boîtier 102 est plus haut que profond.

Les faces avant 102Fr et arrière 102Re ont une longueur (la dimension en X) plus importante que la hauteur (la dimension en Z). Dit autrement, le boîtier 102 est plus long que haut.

Ces considérations de taille s'appliquent similairement au dispositif 100. Le dispositif 100 à une longueur (dimension en X) qui est supérieure à une hauteur (dimension en Z) qui est elle-même supérieure à une profondeur (dimension en Y). Par exemple, la longueur est au moins 3 fois supérieure à la hauteur et la hauteur est 1,5 fois supérieure à la profondeur. Ces dimensions correspondent aux volumes précités pour le dispositif (sous la forme dimension en X, dimensions en Y, dimensions en Z)

Le dispositif 100 comprend un ou plusieurs capteurs physiologiques disposés à différents endroits. Les capteurs physiologiques permettent d'effectuer des mesures physiologiques qui génèrent des données physiologiques.

### Les capteurs physiologiques aux extrémités

En particulier, dans un mode de réalisation, le dispositif 100 comprend un capteur physiologique d'extrémité 106L au niveau de la première extrémité 102L, qu'on nomme premier capteur d'extrémité 106L.

Le premier capteur d'extrémité 106L comprend une surface fonctionnelle 108L. Par surface fonctionnelle 108L, il est signifié une surface destinée à être positionnée face à l'utilisateur, pour interagir avec celui-ci, avec ou sans contact, pour l'obtention de la mesure physiologique par le premier capteur d'extrémité 106L.

Par exemple, le premier capteur d'extrémité 106L peut être un stéthoscope électronique, avec un capteur piézo-électrique et une membrane destinée à être positionnée sur l'utilisateur. La membrane fonctionne comme un amplificateur des ondes mécaniques générées par le coeur ou les poumons. Dans ce cas, la surface fonctionnelle 108L comprend la membrane. La membrane est notamment la partie visible par l'utilisateur du capteur piézo-électrique.

Par exemple, le premier capteur d'extrémité 106L peut être un thermomètre, avec un capteur de type thermopile et une lentille. La lentille peut être entourée d'un cône. Dans ce cas, la surface fonctionnelle 108L comprend la lentille et, le cas échéant, le cône. La lentille et le cône sont notamment les parties visibles par l'utilisateur du thermomètre.

Par exemple, le premier capteur d'extrémité 106L peut être un spiromètre avec un capteur de volume et/ou de débit d'air et un embout buccal. Dans ce cas, la surface fonctionnelle 108L comprend l'embout buccal. L'embout buccal est notamment la partie visible par l'utilisateur du spiromètre.

Le premier capteur d'extrémité 106L est positionné au niveau de la première extrémité 102L et sa surface fonctionnelle 108L est inscrite dans le bord 104L. Cela signifie que, dans une projection selon la direction d'extension X dans un plan transversal YZ au niveau de l'extrémité 102L, la surface fonctionnelle 108L est positionnée à l'intérieur du bord 104L. Formulé autrement, la projection de surface fonctionnelle 108L est comprise dans la projection de la face latérale défini par le bord 106L.

Grâce à ces caractéristiques, la préhension du dispositif 100 n'est pas gênée par le capteur d'extrémité 106L. En particulier, l'utilisateur peut tenir le dispositif 100 par une extrémité, en ayant la main (par exemple la paume) dans le prolongement de la direction d'extension. Cette caractéristique permet aussi de ranger aisément le dispositif 100, par exemple dans une poche ou dans une boîte.

A cet égard, dans un mode de réalisation, la surface fonctionnelle 108L est positionnée à l'intérieur du volume défini par le boîtier 102 (et donc par le bord 104L au niveau de l'extrémité 102L). Le capteur d'extrémité 106L n'est donc pas en saillie hors du boîtier 102. Toutefois, pour certains capteurs, notamment ceux qui nécessitent un contact, comme le stéthoscope, la surface fonctionnelle 108L (par exemple la membrane) peut s'écarter d'au plus 5 mm hors du volume défini par le boîtier 102 le long de la direction d'extension X, voire au plus 2 mm, voire au plus 1 mm.

Dans un mode de réalisation, le dispositif 100 comprend un deuxième capteur physiologique d'extrémité 106R au niveau de la deuxième extrémité 102R. Ce deuxième capteur physiologique 106R est défini de façon similaire au premier capteur physiologique 106L et sera appelé « deuxième capteur d'extrémité ».

Dans un mode de réalisation, illustré sur la figure 1, le premier capteur d'extrémité 106L est un stéthoscope électronique et le deuxième capteur d'extrémité 106R est un capteur de température.

Dans un mode de réalisation, illustré sur les figures 7 à 9 et qui seront décrites plus tard, le premier capteur d'extrémité 106L est un capteur de température et le deuxième capteur d'extrémité 106R est un stéthoscope électronique.

Le positionnement des deux capteurs d'extrémité 106L, 106R permet d'effectuer ces deux mesures, dans deux positions différentes qui vont être décrites dans les paragraphes suivants, avec une maniabilité accrue et une préhension qui ne soit pas gênée par les capteurs d'extrémité 106L, 106R lors de la manipulation du dispositif par l'utilisateur pour effectuer les mesures.

Le dispositif 100 comprend en outre un ou plusieurs capteurs physiologiques de doigt 110L, 110R disposé(s) sur le boîtier 102 (appelé « capteur de doigt » par la suite). Chaque capteur de doigt 110L, 110R est destiné à recevoir un doigt de l'utilisateur (index ou pouce par exemple). Plusieurs modes de réalisation seront décrits par la suite.

Le dispositif 100 comprend un affichage 112, par exemple un écran (illustré en pointillé sur la figure 1 car le contour de l'écran est invisible pour l'utilisateur, ou a *minima* peu visible, dans ce mode de réalisation), destiné à afficher des informations et/ou des résultats de mesure pour l'utilisateur.

Dans un mode de réalisation, l'affichage 112 est configuré pour afficher des informations selon une direction de lecture parallèle et/ou transversale à la direction d'extension X. Dans un mode de réalisation, le dispositif comprend un gyromètre et/on accéléromètre configuré pour déterminer l'orientation dans l'espace du dispositif 100 et adapter la direction de lecture de l'affichage 112 en fonction de cette orientation. Ainsi, comme il sera expliqué plus en détail par la suite, l'affichage peut être transversal lorsque l'utilisateur tient le dispositif à une main et longitudinal lorsque l'utilisateur tient le dispositif à deux mains.

Dans un mode de réalisation, l'affichage 112 est positionné sur la face avant 102Fr du boîtier 102, de sorte que l'utilisateur puisse voir l'affichage 112 en position de navigation, en position à une main et en position à deux mains. Le positionnement de l'affichage 112 sera décrit plus en détail par la suite.

Le dispositif 100 comprend en outre une interface physique 114 avec l'utilisateur, qui peut prendre la forme d'un joystick (anglicisme de "bouton de navigation" en français), de flèche, etc. L'interface physique 114 est fonctionnellement reliée à l'affichage 112 et permet par exemple de naviguer dans un menu affiché sur l'affichage 112 et de sélectionner des actions. L'interface physique 114 peut être positionné sur la face avant 102Fr du boîtier 102.

Pour simplifier la navigation, l'affichage 112 et l'interface physique 114 sont positionnés côte à côte, par exemple sur la face avant 102Fr. Dans le mode de réalisation des figures 1 à 10, l'interface physique 114 est positionnée du côté de la deuxième extrémité 102R.

La figure 3 illustre la position 300, dite position de navigation. La position de navigation est préférentiellement une position de navigation à une main. Il s'agit d'une position durant laquelle l'utilisateur utilise l'interface physique 114 pour sélectionner une mesure. Par exemple, pour le mode de réalisation illustré, l'utilisateur saisit le dispositif 100 dans sa main droite ou gauche, comme une télécommande de télévision, en coinçant le boîtier entre la paume 304 et les doigts 306. Au vu de l'arrangement entre l'affichage 112 et l'interface physique 114, la deuxième extrémité 102R est arrangée vers le bas et la première extrémité 102L est arrangée vers le haut. De la sorte, le pouce 308 trouve naturellement l'emplacement de l'interface physique 114. Cette position convient de manière égale aux gauchers et aux droitiers. Dans cette position 300, l'affichage 112 peut être configuré pour afficher des informations selon une direction de lecture transversale par rapport à la direction d'extension X. Comme illustré ici, le menu de sélection des mesures disponibles (ECG, SpO2, Stetho, Thermo, par exemple) est par exemple affiché. L'utilisateur peut alors naviguer puis sélectionner la mesure qu'il désire effectuer avec l'interface physique 114.

La figure 4 illustre la position 400, dite position de manipulation à une main. En particulier, il s'agit d'une position de manipulation à une main pour le premier capteur d'extrémité 106L au niveau de la première extrémité 102L. Dans cette position 400, l'utilisateur tient le dispositif 100 entre le pouce et l'index (main gauche ou droite) et vient mettre la surface fonctionnelle 108L en face du corps de l'utilisateur (qui est généralement le manipulateur aussi). Dans le cas du mode de réalisation des figures 1 à 6, le premier capteur 106L est un stéthoscope, la surface fonctionnelle 108L est la membrane et la partie du corps est le torse 402. La surface fonctionnelle 108L est alors en contact avec le corps. Dans cette position 400, l'affichage 112 peut être configuré pour afficher des informations selon une direction de lecture transversale par rapport à la direction d'extension X. Comme illustré ici, les informations peuvent être une représentation temporelle des sons captés par le stéthoscope ou bien des informations relatives au placement du stéthoscope sur le corps. L'utilisateur peut alors visualiser la mesure en cours et ajuster la position du dispositif pour améliorer le volume des sons captés.

La figure 5 illustre la position 500, dite position de manipulation à une main. En particulier, il s'agit d'une position de manipulation à une main pour le deuxième capteur d'extrémité 104R au niveau de la première extrémité 102R. Dans cette position 400, l'utilisateur tient le dispositif 100 entre le pouce et l'index et vient mettre la surface fonctionnelle 108R en face du corps. Dans le cas du mode de réalisation de la figure 1, le deuxième capteur 106R est un thermomètre, la surface fonctionnelle 108R est une lentille et/ou un cône et la partie du corps est le front 502. Le dispositif 100 vient alors en face du front 502, sans contact. En variante, le dispositif 100 peut venir en contact avec le front pour mesurer la température. Dans cette position 500, l'affichage 112 peut être configuré pour afficher des informations selon une direction de lecture transversale par rapport à la direction d'extension X. Comme illustré ici, le résultat de la mesure de température peut être affiché.

### Le capteur de doigt

Comme indiqué précédemment, le dispositif 100 comprend un premier capteur de doigt 110L, positionné à proximité de la première extrémité 102L afin qu'en position de préhension à deux mains le capteur de doigt 110L soit positionné sous un doigt. Une telle position 600 est illustrée en figure 6.

Ce capteur de doigt 100L peut comprendre une électrode, par exemple une électrode ECG et/ou une électrode d'impédancemétrie pour analyse d'impédance (par exemple impédancepléthysmogramme IPG ou composition corporelle), un capteur optique (par exemple de type PPG, photopléthysmogramme, ou laser), un capteur de force, un capteur de pression, un capteur magnétique, etc.

Ce premier capteur de doigt 100L peut être arrangé à différents emplacements du boîtier 102.

En relation avec les figures 1 à 6, le capteur de doigt 110L est positionné pour que l'index 601L, 601R de l'utilisateur se positionne naturellement dessus lorsque le dispositif est tenu par la première extrémité 102L et/ou la deuxième extrémité 102R. A cet égard, le capteur de doigt 110L est positionné sur la face supérieure 102To.

Selon un mode de réalisation non illustré, le capteur de doigt est positionné sur le boîtier 102 pour tomber sous le pouce lorsque le dispositif est tenu par la première extrémité 102L et/ou la deuxième extrémité 102R. A cet égard, le capteur de doigt est positionné sur la face avant 102Fr. Par exemple, le capteur de doigt peut être intégré à l'interface physique 114 pour simplifier l'interface utilisateur : sur la figure 6, il est visible que l'utilisateur peut simplement étendre le pouce pour atteindre l'interface physique ou bien le capteur peut être positionné au voisinage de l'interface physique 114.

Alternativement, le capteur de doigt est positionné sur la face avant 102Fr, espacé de l'interface physique 114.

Selon un mode de réalisation illustré à la figure 10 et qui sera décrit par la suite, le capteur de doigt est une électrode et l'électrode est disposé sur tout ou partie du bord 104L, 104R de l'extrémité 102L, 102R, par exemple via un dépôt métallique ou un ajout d'une pièce métallique.

Dans un mode de réalisation, le dispositif 100 comprend un deuxième capteur de doigt 110R positionné à proximité de la deuxième extrémité 102R afin qu'en position 600, de manipulation à deux mains, chaque capteur de doigt 110L, 110R soit positionné sous un doigt.

Selon un mode de réalisation, « à proximité d'une extrémité » signifie « entre le bord de l'extrémité et strictement la moitié de la longueur L depuis le bord ». Selon un autre mode de réalisation, « à proximité » signifie « entre le premier bord 104L et strictement un quart de la longueur L depuis le premier bord 104L ». Ces exemples sont visibles en figure 2, avec les distances U2 et L/4 représentées.

Dans un mode de réalisation, le premier capteur de doigt 110L et le deuxième capteur de doigt 110R sont des électrodes, par exemple des électrodes ECG (électrocardiogramme). Le deuxième capteur de doigt 110L peut être positionné de façon similaire au premier capteur de doigt 110R tel que décrit précédemment, c'est-à-dire que les deux capteurs de doigt sont sur la face supérieure 102To (symétrie de positionnement visible sur la face supérieure 102To en figure 2), ou bien que les deux capteurs de doigt sont sur la face avant 102Fr. Plus généralement, le premier capteur de doigt 110L et le deuxième capteur de doigt 110R peuvent être alignés parallèlement à la direction d'extension X. La symétrie de positionnement permet de simplifier la prise de mesure. Alternativement, un des deux capteurs de doigt 110L, 110R est sur la face supérieure 102To et l'autre des deux capteurs de doigt 110L, 110R est sur la face avant 102Fr. En touchant simultanément les deux électrodes ECG avec deux doigts de mains différentes, l'utilisateur peut ainsi réaliser un ECG.

Dans ce mode de réalisation, les deux électrodes peuvent être espacées, le long de la direction d'extension X, d'au moins 5 cm. Cette distance permet d'assurer une bonne manipulation sans risque d'avoir les mains qui se touchent.

Dans un mode de réalisation, uniquement deux électrodes ECG peuvent être prévus. En s'affranchissant de la troisième électrode souvent prévue pour réaliser un ECG, la manipulation du dispositif 100 est grandement simplifiée, puisque la contrainte de positionnement ne porte que sur les doigts, qui tombent naturellement en position.

La figure 6 illustre la position 600 de mesure à deux mains dans laquelle au moins un capteur de doigt 110L, 110R est utilisé. Cette position consiste à tenir le dispositif 100 par les extrémités 102L, 102R, avec respectivement les mains 602L, 602R. En position 600, les phalanges proximales 604L, 604R des index 601L, 601R se retrouvent dans le prolongement de la direction d'extension X, tandis que la face arrière 102Re repose sur les majeurs ou annulaires 606L, 606R et que les pouces 608L, 608R viennent s'appuyer sur la face avant 102Fr.

Lorsque l'utilisateur tient le dispositif 100 en position 600 de manipulation à deux mains, les doigts se placent naturellement sur les capteurs de doigt 110L, 110R. De plus, les positionnements des capteurs de doigt assurent aussi que l'affichage 112 demeure bien visible pendant la manipulation pour que l'utilisateur puisse recevoir des informations en temps réel.

Dans cette position 600, l'affichage 112 peut être configuré pour afficher des informations selon une direction de lecture parallèle à la direction d'extension X. Comme illustré ici, une représentation temporelle de l'ECG en cours de mesure peut être affichée. L'utilisateur peut alors visualiser la mesure en cours.

En outre, le ou les capteurs de doigts 110L, 110R, qu'ils soient sur la face supérieure 102To ou la face principale 102Fr, sont aisément atteignables en position 600 du fait de l'intégration du ou des capteurs d'extrémité 106L, 106R dans les extrémités 102L, 102R (en étant inscrits dans le bord). Aucun des capteurs d'extrémité 106L, 106R ne génèrent d'encombrement additionnel (ou seulement marginal) par rapport au boîtier 102 et n'interfère pas avec la main lors de la position 600. Le dispositif 100 peut donc être basculé rapidement et aisément entre les positions 300, 400, 500 à une main et la position 600 à deux mains.

Dans un mode de réalisation, le dispositif 100 comprend un troisième capteur de doigt 116L positionné à proximité de la première extrémité 102L (comme illustré sur les figures 1 et 2) ou de la deuxième extrémité 102R afin qu'en position de préhension à deux mains le troisième capteur de doigt 116L sous positionné sous un doigt. Par exemple, le troisième capteur de doigt 116L est un capteur optique intégré à l'électrode (par exemple une électrode ECG, afin de faire les mesures d'ECG et optique en même temps). Une telle intégration permet d'avoir une même position de doigt pour plusieurs mesures.

Dans un mode de réalisation, le dispositif 100 comprend un quatrième capteur de doigt (non visible car positionné sous le capteur optique), situé de la même façon à proximité de la première extrémité ou de la deuxième extrémité. Le quatrième capteur de doigt peut être un capteur de force, qui mesure une force que le doigt applique sur le dispositif 100. Par exemple, le capteur de force est situé sous le capteur optique.

Dans un mode de réalisation, le ou les capteurs de doigt 110L, 110R, 116L sont positionné(s) à l'intérieur d'un volume défini par le boîtier 102. Formulé autrement, ils ne s'étendent pas, hors du boîtier 102, ou alors faiblement, par exemple sur une distance de moins de 2 mm, ou, dans le cadre d'un capteur de doigt 110L, 110R, 116L dans l'interface physique 114, en saillie par rapport à l'interface physique 114. Cet arrangement fait que les capteurs de doigt 110L, 110R, 116L ne gênent pas la manipulation lors des positions 300, 400 et 500, pour lesquelles le capteur de doigt 110L, 110R, 116L n'est pas utilisé.

Ainsi, le dispositif 100 permet donc au moins deux positions de manipulation : une position de navigation à une main 300, une ou deux positions 400, 500 de manipulation à une main et une position 600 de manipulation à deux mains. En deux positions de mesure, il est ainsi possible d'effectuer au moins trois mesures, voire quatre ou cinq.

### Variante pour le stéthoscope

Dans le mode de réalisation illustré sur les figures 1 à 6, l'affichage 112 est disposé entre l'interface de navigation 114 et le premier capteur 106L, qui est un stéthoscope (le long de la direction d'extension X). Ainsi, en position 400, c'est-à-dire en position de manipulation à un main avec le premier capteur d'extrémité 106L, qui est ici un stéthoscope, l'affichage 112 se trouve proche du corps, ce qui peut gêner la lecture de l'affichage.

Les figures 7 à 9 illustrent un dispositif 700, variante du dispositif 100 du mode de réalisation de la figure 1. Dans le dispositif 700, le deuxième capteur d'extrémité 102R est un stéthoscope. Le premier capteur d'extrémité 102L peut alors être un thermomètre. Le reste du dispositif 700 est inchangé par rapport à celui des figures 1 à 6. Dans ce mode de réalisation, l'interface physique 114 se trouve entre l'affichage 112 et le deuxième capteur d'extrémité 106R qui est un stéthoscope (le long de la direction d'extension X).

De la sorte, le long de la direction d'extension X, l'interface physique 114 se retrouve entre l'affichage 112 et le stéthoscope 106R (qui est le deuxième capteur d'extrémité 106R). La figure 9 représente une position 900, correspondant à la position 400 précédemment décrite en référence à la figure 4, dans laquelle l'utilisation est en position à une main pour le stéthoscope.

Du fait du positionnement de l'interface physique 114, l'affichage 112 se retrouve davantage éloigné du deuxième bord 104R (distance D sur la figure 9 supérieure à la distance D, non représentée, de la figure 4). Ainsi, lorsque le deuxième capteur 106R (ici le stéthoscope) est au contact du torse 402, l'affichage 112 est davantage éloigné du torse 402 quand dans le mode de réalisation des figures 1 à 6, ce qui facilite sa lecture en limitant l'angle de pliage de la nuque et en permettant aussi à ceux qui ont une masse grasse plus élevée ou une pilosité plus élevée de ne pas masquer partiellement l'affichage 112.

En particulier, lors de la mesure du stéthoscope, l'affichage 112 peut indiquer des directives à l'utilisateur pour le placement de la membrane 108R. Il est donc important que l'utilisateur puisse aisément voir l'affichage 112.

De plus, la partie du boîtier 102 entre la deuxième bord 104R et l'interface physique 114 permet de tenir le dispositif 700, comme illustrée dans la position 800 de navigation, qui est identique pour cette variante.

L'utilisateur tient ainsi le dispositif 700 comme en position 800, illustrée en figure 8 (position similaire à la position 300 de la figure 3). Avec l'interface physique 114, il sélectionne la mesure stéthoscope puis il n'a plus qu'à venir déplacer le stéthoscope directement contre son torse 402, en tenant le dispositif 700 entre le pouce et l'index/majeur, pour l'amener en position 900 illustrée en figure 9. La transition entre la position 800 et la position 900 est particulièrement simple et ne nécessite pas de rotation du dispositif 700 dans la main. De plus, comme indiqué précédemment, l'affichage 112 est plus facilement visible dans cette variante grâce à la distance D.

Dans cette variante, lorsque le premier capteur d'extrémité 102L est un thermomètre, la position 500 de la figure 5 peut légèrement changer, dans la mesure où la main tient le dispositif 700 par le côté opposé au thermomètre 102L. Par conséquence, en montant la main au front, le dispositif 700 peut se retrouver avec la face 102Fr regardant vers le bas, mais la mise en position demeure simple, avec aucune rotation du dispositif 700 dans la main.

### Variante du dispositif

La figure 10 illustre un autre mode de réalisation d'un dispositif 1000. Ce mode de réalisation est similaire au dispositif 100, à l'exception d'un capteur d'extrémité 1006L, qui n'est pas inscrit dans un bord comme précédemment décrit. Les positions d'utilisation 300, 500 et 600 demeurent inchangées.

Sur la figure 10, il s'agit du premier capteur d'extrémité 1006L qui est modifié, mais ce pourrait être le deuxième capteur d'extrémité 1006R.

Dans ce mode de réalisation, un capteur physiologique d'extrémité 1006L comprend une face fonctionnelle 1008L qui est disposée sur la face avant 102Fr ou la face arrière 102Re. Dans l'exemple illustré, la surface fonctionnelle 1008L est positionné sur la face arrière 102Re, du côté opposé à l'affichage 112 et l'interface physique 114. En particulier, ce capteur 1006L est un stéthoscope et l'interface fonctionnelle 1008L est une membrane. La membrane s'étend typiquement selon un plan orthogonal YZ (appelé « plan de membrane ») la direction d'extension X

Toutefois, le capteur d'extrémité 1006L demeure à proximité de l'extrémité 1002L, avec notamment « à proximité » qui signifie dans la moitié ou le quart de la longueur L du dispositif 1000 (même définition que précédemment).

L'interface physique 114 est située sur la face avant 102Fr, entre la deuxième extrémité 102R et la moitié de la longueur L, voire un tiers de la longueur L.

L'utilisateur peut tenir le boîtier 102 entre l'interface physique 114 et le deuxième bord 104R pour appliquer la membrane 1008L sur le torse. En position d'utilisation du stéthoscope, la direction d'extension X est donc essentiellement parallèle au torse, alors qu'en position 300 du dispositif 100, la direction d'extension X est essentiellement perpendiculaire au torse.

La figure 10 permet d'illustrer un autre mode de réalisation concernant le capteur de doigt, qui a été mentionné précédemment. Ce mode de réalisation est sans lien direct avec le stéthoscope précédemment décrit.

En effet, un ou plusieurs capteurs de doigt 1010R (seul le capteur de doigt 1010R est concerné sur la figure 10 mais en variante les deux capteurs de doigts 1010R, 1010L peuvent être concernés) peut être positionné sur tout ou partie du bord 104R de l'extrémité 102R. Ainsi, le contact ne s'effectue plus uniquement par le doigt mais aussi par la paume de la main, en position de manipulation à deux mains, comme en position 600.

Le capteur peut être réalisé par un dépôt métallique sur le bord ou par ou un ajout d'une pièce métallique.

Ce mode de réalisation se prête particulièrement au cas où le capteur de doigt est une électrode. Dans le cas d'un capteur optique, le positionnement sous le doigt reste préférable.

### Variantes

Comme cela a été décrit, les combinaisons sont possibles entre les variantes et modes de réalisation données.

En particulier, le premier capteur d'extrémité tel que décrit peut être au niveau de la deuxième extrémité et, le cas échéant, le deuxième capteur d'extrémité tel que décrit peut être au niveau de la première extrémité.

### Présentation du dispositif et de son environnement

La figure 11 illustre un schéma de l'architecture d'un dispositif 100, 700, 1000 (référencé 1100 sur cette figure) tel que décrit et de son environnement.

Le dispositif 1100 comprend une unité de contrôle 1102 avec une circuiterie de contrôle 1104 comprenant un processeur 1106, une mémoire 1008 et une interface I/O 1110 (« In/Out » en anglais ou « Entrée/Sortie » en français) pour communiquer avec les autres composants.

La mémoire 1008 stocke des programmes, instructions ou autres permettant à la fois la navigation sur le dispositif 1100 ainsi que la prise des mesures (algorithmes notamment). La mémoire 1008 en particulier se décompose en une mémoire volatile, de type RAM, et une mémoire non-volatile, de type flash (ou ROM ou SSD).

Le dispositif 1100 comprend un ou plusieurs capteurs 1112 (tous les capteurs décrits précédemment sont schématisés sous une seule référence 1112).

L'unité de contrôle 1102 comprend typiquement un module d'interface 1114 faisant interface entre les capteurs 1112 et l'interface I/O 1110 de la circuiterie de contrôle 1104. Le module d'interface 1114 comprend notamment des ADC, des filtres, des amplificateurs, etc.

Le dispositif 1100 comprend en outre un affichage 1116, qui communique avec l'interface I/O 1110, et une interface mécanique 1118 qui communique avec le module d'interface 1114 pour la navigation dans le menu de l'affichage 1116.

Pour alimenter les différents composants en énergie électrique, le dispositif 1100 comprend une batterie 1120, par exemple une pile ou une batterie rechargeable. La batterie 1120 est configurée pour alimenter notamment l'unité de contrôle 1104, l'affichage 1114 et les capteurs 1112.

Enfin, pour la connectivité, le dispositif 1100 comprend un module de communication sans fil 1122 (Bluetooth, BLE, Wifi, cellulaire, etc.), relié à la circuiterie de contrôle 1102. Le module 1122 permet de communiquer, via un réseau de communication 1124 à un terminal mobile 1126 (par exemple un téléphone portable de type smartphone) et/ou un serveur distance 1128. Les données physiologiques ainsi acquises par le dispositif 1100 peuvent être stockées, analysées, traitées dans le serveur 1128 et affichées par le terminal mobile 1126. Le terminable mobile 1126 peut aussi servir de relai entre le dispositif 1100 et le serveur 1128 (par exemple dans le cas d'une communication Bluetooth ou BLE).

## Revendications

1. Dispositif portable de mesure physiologique (700) préhensible par un manipulateur comprenant :
- un boîtier (102) de forme allongée selon une direction d'extension (X) et comprenant, selon la direction d'extension, une extrémité (102R),
- un stéthoscope électronique (106L, 106R) positionné au niveau de l'extrémité (102R) et comprenant une membrane (108R) s'étendant dans un plan de membrane (YZ),
- un affichage (112), arrangé sur le boîtier (102), l'affichage (112) s'étendant selon une direction d'extension (X) orthogonal au plan de membrane (YZ),
- une interface physique (114), reliée fonctionnellement à l'affichage (112) et positionnée sur le boîtier (102) entre l'affichage (112) et la membrane (108R) le long de la direction d'extension.

2. Dispositif portable selon la revendication précédente, dans lequel l'affichage (112) est configuré pour afficher des informations lisibles depuis un sens transversal à la direction d'extension.

3. Dispositif portable selon l'une quelconque des revendications précédentes, dans lequel l'extrémité (102R) définit un bord (104R) et dans lequel la membrane (108R) est inscrite dans le bord (104L, 104R) au niveau de l'extrémité (102L, 102R).

4. Dispositif portable selon l'une quelconque des revendications précédentes, dans lequel le plan de membrane s'étend orthogonalement à la direction d'extension (X).

5. Dispositif portable selon l'une quelconque des revendications précédentes, dans lequel le dispositif comprend une face (102Fr), dite face avant, et dans lequel l'affichage (112) et l'interface physique (114) sont positionnés sur la face avant (102Fr).

6. Dispositif portable selon l'une quelconque des revendications précédentes, dans lequel l'extrémité (102R) définit un bord (104R), l'interface physique étant positionnée entre le bord (104R) et la moitié, voire un tiers, d'une longueur (L) du dispositif le long de la direction d'extension, depuis le bord (104R).

7. Dispositif portable selon l'une quelconque des revendications, dans lequel l'extrémité (102R) est une deuxième extrémité et le boîtier (102) comprend une première extrémité (102L) opposée à la deuxième extrémité (102R) le long de la direction d'extension (X), le dispositif comprenant une forme essentiellement parallélépipédique avec une face avant (102Fr) et une face arrière (102Re) entre la première extrémité (102L) et la deuxième extrémité (102R), dans lequel l'affichage (112) et l'interface physique (114) sont positionnés sur la face avant (102Fr).

8. Dispositif selon l'une quelconque des revendications précédentes, dans lequel l'extrémité (102R) est une deuxième extrémité et le boîtier (102) comprend une première extrémité (102L) opposée à la deuxième extrémité (102R) le long de la direction d'extension (X), le dispositif comprend en outre un capteur d'extrémité (102L) disposé au niveau de la première extrémité (102L).

9. Dispositif selon la revendication 8, dans lequel le capteur d'extrémité (102L) est un thermomètre.

10. Dispositif portable selon l'une quelconque des revendications précédentes, dans lequel l'affichage (112) est configuré pour afficher des indications relatives au placement du stéthoscope sur le corps.

11. Dispositif selon l'une quelconque des revendications précédentes, présentant un volume inférieur à 20x5x10cm.

12. Dispositif selon l'une quelconque des revendications précédentes, présentant un volume inférieur à 15x2,5x4cm.

13. Procédé d'utilisation du dispositif selon l'une quelconque des revendications précédentes comprenant au moins les étapes suivantes :
- utilisation de l'interface physique du dispositif dans une position de navigation manipulation à une main (300),
- mesure avec le stéthoscope dans une position de manipulation à une main (400).
